# FASCICULE DE BREVET EUROPEEN

(11) **EP 0 753 346 B1**
(45) Date de publication et mention de la délivrance du brevet: **06.09.2000**
(21) Numéro de dépôt: 96401416.1
(22) Date de dépôt: 26.06.1996
(51) Int. Cl.: B01J 31/14, C07C 2/36

(54) **Nouvelle composition catalytique pour catalyse biphasique, en particulier à base de complexes du nickel et procédé pour l'oligomérisation des oléfines**
Neue katalytische Zusammenstellung zur Zwei-Phasen-Katalyse, insbesondere auf Grund von Nickelkomplexen, und Verfahren zur Oligomerisierung von Olefinen
New catalytic composition, particularly based on nickel complexes, for two-phase catalysis, and a process for olefin oligomerisation

(30) Priorité: 11.07.1995 FR 9508492
(43) Date de publication de la demande: 15.01.1997
(73) Titulaire: INSTITUT FRANCAIS DU PETROLE, 92502 Rueil-Malmaison (FR)
(72) Inventeur: Chauvin, Yves, 78230 Le Pecq (FR); De Souza, Roberto, 90460 Porto Alegre (BR); Olivier, Hélène, 92500 Rueil Malmaison (FR)

(56) Documents cités:
- EP-A- 0 009 035
- EP-A- 0 470 794
- EP-A- 0 531 174
- US-A- 4 187 197
- US-A- 4 996 273

## Description

Un objet de la présente invention est une nouvelle composition catalytique et son utilisation pour la dimérisation, la codimérisation, l'oligomérisation et la co-oligomérisation des oléfines la dite composition comprenant au moins un halogénure de lithium, au moins un halogénure d'hydrocarbylaluminium et au moins d'un composé d'un élément catalytique et en particulier un composé du nickel.

On connaît un grand nombre de systèmes catalytiques à base de composés du nickel qui sont actifs pour l'oligomérisation des oléfines. Ces catalyseurs peuvent être de type hétérogène, c'est-à-dire qu'ils sont totalement insolubles dans les composés organiques compatibles ou dans les oléfines et leurs produits d'oligomérisation; à titre d'exemples on peut citer le sulfate de nickel déposé sur une alumine, le chlorure de nickel déposé sur silice-alumine, les zéolithes échangées au nickel. Ces catalyseurs peuvent être de type homogène, c'est-à-dire dissous dans un solvant organique tel que le chlorobenzène ou encore dissous dans les produits d'oligomérisation. Ces catalyseurs peuvent être mis en oeuvre dans des organochloroaluminates à base de sels d'ammonium quaternaires constituant ainsi un système biphasique liquide-liquide comme décrit dans le brevet FR 2.659.871.

Tous ces catalyseurs présentent des inconvénients inhérents à leur mise en oeuvre. Les catalyseurs hétérogènes classiques ne permettent pas de bénéficier de "l'effet phosphine" tel que décrit par G.Wilke *et al.* dans Angewante Chemie, Edition Internatinale 1966, 5, 151, et présentent des limitations dues aux transferts de masse (pertes de sélectivité) ou de chaleur. Les catalyseurs homogènes posent des problèmes d'une mauvaise utilisation du catalyseur qui quitte le réacteur étant encore actif, et de leur séparation des produits.

Il a de plus été trouvé qu'un mélange liquide d'au moins un halogénure de lithium avec au moins un halogénure d'hydrocarbylaluminium, dans lequel a été introduit au moins un élément catalytique et au moins une phase hydrocarbonée, peut se transformer en un catalyseur solide qui se sépare de la phase hydrocarbonée.

La composition catalytique liquide, mélange liquide plus élément catalytique, présente au moment du début d'introduction de la phase hydrocarbonée, catalytiquement active dans une catalyse biphasique liquide-liquide, peut donc se transformer plus ou moins rapidement selon les conditions opératoires, en présence d'au moins une phase liquide hydrocarbonée, qui est avantageusement le milieu réactionnel, en une composition catalytique solide. De façon extrêmement surprenante, cette composition catalytique solide conserve son activité catalytique. La catalyse biphasique devient alors solide-liquide.

Un autre objet de l'invention est une composition catalytique solide ainsi obtenue.

La phase hydrocarbonée contient avantageusement le réactif, (l'oléfine), et/ou au moins le produit de la réaction, (l'oligomère). La phase organique peut être introduite avant, pendant ou après l'élément catalytique, mais de préférence l'élément catalytique est apporté avant la phase hydrocarbonée ou simultanément.

Ainsi les composés de nickel dissous dans le mélange liquide que font les halogénures de lithium avec les halogénures d'hydrocarbylaluminium constituent, en présence des produits d'oligomérisation et/ou en présence d'oléfines, un catalyseur solide hétérogène-dispersé actif pour l'oligomérisation ou la co-oligomérisation des mono-oléfines.

Un autre objet de l'invention est un procédé pour l'oligomérisation et la co-oligomérisation des oléfines , procédé dans lequel la ou les oléfines sont mises au contact d'un composé du nickel dissous au moins en partie dans le mélange liquide que font les halogénures de lithium avec les halogénures d'hydrocarbylaluminium.

Les halogénures d'hydrocarbylaluminium selon l'invention ont pour formule générale Al₂XₓR₆₋ₓ dans laquelle X est le chlore ou le brome, R est un reste alkyl, cycloalkyl, aryl ou aralkyl comportant de 1 à 10 et de préférence 2 à 6 atomes de carbone, x prenant les valeurs de 2, 3 ou 4. Ils peuvent être utilisés seuls ou en mélange. A titre d'exemples on peut citer les chlorures d'alkylaluminium tels que le dichlorure d'éthylaluminium, le dichlorure d'isobutylaluminium, le sesquichlorure d'éthylaluminium, le chlorure de diéthylaluminium.

Les halogénures de lithium selon l'invention sont le chlorure et/ou le bromure de lithium.

Selon un mode préféré de l'invention on mélange séparément l'halogénure de lithium, préalablement séché par chauffage, avec l'halogénure d'hydrocarbylaluminium, à l'état pur ou en présence d'un hydrocarbure. Les composés sont mis en oeuvre dans un rapport molaire Al/Li tel que le mélange soit liquide au moment de l'introduction de l'oléfine. De préférence ce rapport est supérieur à 1 et avantageusement compris entre 1 et 4, de préférence entre 1,5 et 3 pour une mise en oeuvre à une température comprise entre 0 et 50°C. On cherche généralement à obtenir un mélange qui soit liquide entre -50 et +100°C.

Les composés de l'élément catalytique, et en particulier du nickel selon l'invention sont de préférence des complexes zérovalents, monovalents ou divalents, ces derniers pouvant contenir ou non une ou deux liaisons nickel-carbone. Les composés particulièrement souhaitables selon l'invention sont, entre autres, les sels de nickel bivalents tels les halogénures, le sulfate, le phosphate, les carboxylates, les acétylacétonates, les tétrahalogénoborates et les complexes que font ces sels avec les amines, les phosphines, les ethers, les nitriles, les esters, seuls ou en mélange et avantageusement le mélange décrit dans la demande FR 93/11 382. On peut également ajouter des phosphines aux sels qui n'en comportent pas. A titre d'exemples on peut citer NiCl₂, NiCl₂.2Pyridine, NiCl₂.2P(i-Pr)₃, NiCl₂.2P(Bu)₃, Ni(acétonitrile)₆(BF₄)₂, le 2-éthylhexanoate de nickel, l'acétylacétonate de nickel.

Le composé de nickel peut être introduit avec un diluant, par exemple un hydrocarbure, de préférence un hydrocarbure aromatique.

Le mélange du composé de l'élément catalytique (du nickel) avec la composition liquide obtenue précédemment se fait de préférence en présence d'une oléfine, par exemple l'oléfine dont on veut catalyser l'oligomérisation, en présence ou non d'un autre hydrocarbure.

La concentration du (des) composé(s) de l'élément catalytique (nickel) dans le mélange liquide formé par au moins un halogénure de lithium et au moins un halogénure d'hydrocarbylaluminium est avantageusement comprise entre 1 mmole par litre et 500 mmoles par litre, de préférence entre 2 et 200 mmoles par litre, et encore entre 2 et 100, voire 2 à 50.

Les oléfines auxquelles le procédé peut s'appliquer sont, par exemple, l'éthylène, le propylène, les n-butènes, les n-pentènes, seuls ou en mélange.

Dans le procédé d'oligomérisation, la ou les oléfines pourront être utilisées pures ou diluées par des hydrocarbures saturés tels que ceux qu'on trouve dans les "coupes" issues des divers procédés de raffinage des hydrocarbures, comme par exemple l'éthane avec l'éthylène, le propane avec le propylène, les butanes avec les butènes .

La température à laquelle se fera l'oligomérisation sera comprise entre -30°C et 100°C, de préférence entre -10°C et 50°C. La pression peut être comprise entre la pression atmosphérique ou une pression inférieure à la pression atmosphérique et 10 MPa, de préférence entre la pression atmosphérique et 1 MPa, mais sera suffisante pour maintenir au moins en partie, la ou les oléfines en phase liquide.

La réaction catalytique d'oligomérisation des oléfines, peut être conduite en système fermé, en système semi-ouvert ou en continu avec un ou plusieurs étages de réaction. A la sortie du réacteur la phase hydrocarbonée est séparée de la phase solide puis lavée à l'eau. La phase solide qui contient le catalyseur et qui est dans le réacteur est réutilisée.

Les exemples suivants illustrent l'invention sans en limiter la portée:

### EXEMPLE 1

### Préparation du mélange liquide chlorure de lithium-dichlorure d'éthylaluminium

Dans un ballon de 100 mL muni d'une entrée d'argon et d'un barreau magnétique on introduit 2,55 g (0,06 mole) de chlorure de lithium, préalablement séché par chauffage sous vide. Le ballon est refroidi à -30°C et on introduit 14 mL (0,14 mole) de dichlorure d'éthylaluminium. Le système est réchauffé lentement jusqu'à la température ambiante et mis sous agitation. Après 2 heures on obtient un mélange liquide incolore.

### Oligomérisation du propylène

Dans un réacteur en verre d'une contenance de 150mL, muni d'un système d'agitation et d'une double enveloppe pour la circulation d'un fluide caloporteur, purgé d'air et d'humidité, on a introduit à 0°C, sous pression atmosphérique de propylène, 64mg (0,13 mmole) de bis-tétrafluoroborate d'hexaquis acétonitrile nickel (II), 10 mL d'heptane et, 4 mL ( 17 mmoles basé sur le lithium) de la composition précédente. Le mélange forme deux phases liquides non miscibles. On règle la température du réacteur à 5°C et on suit la consommation de propylène par la perte de poids du réservoir d'alimentation qui est relié au réacteur par un détendeur. Les produits obtenus sont soutirés périodiquement et analysés. Dès le premier soutirage après une heure de réaction la composition liquide inférieure se transforme en un solide jaune. Après une période initiale de faible consommation de propylène on atteint une productivité de 4,8 kg/g nickel/heure. Les produits obtenus au quatrième soutirage ont la composition suivante :

| Sélectivités (%) | | Sélectivité dans la Fraction C6 (%) | |
|---|---|---|---|
| C6 | 84 | Méthyl-2-Pentène-1 | 4 |
| C9 | 15 | Méthyl-2-Pentène-2 | 40 |
| C12+ | 1 | Méthyl-4-Pentène-1 | 1 |
| | | Méthyl-4-Pentène-2 | 30 |
| | | Hexènes | 16 |
| | | Diméthyl-2,3-Butène-1 | 4 |
| | | Diméthyl-2,3-Butène-2 | 5 |

### EXEMPLE 2

On a opéré comme dans l'exemple précédent à ceci près qu'on a opéré à 10°C, sous pression atmosphèrique de butène-1 et on a suivi la consommation de butène-1 au cours du temps. Après une période initiale de faible consommation de butène on atteint une productivité de 3,0 kg/g nickel/heure. Dès le premier soutirage la composition inférieure se transforme en un solide jaune. Les produits obtenus sont des dimères (isooctènes) 95±2%, des trimères (C₁₂) 5±2% ainsi que faibles quantités de produits plus lourds (C₁₆₊). Les produits dans la fraction C8 constituent un mélange complexe d'oléfines qui, après hydrogénation, s'avèrent être le n-octane (6%), méthyl-3-heptane (58%) et diméthyl-3,4-hexane (36%).

## Revendications

1. Composition catalytique comprenant au moins un halogénure de lithium, au moins un halogénure d'hydrocarbylaluminium et au moins un composé d'un élément catalytique.

2. Composition selon la revendication 1, dans laquelle le composé est un composé du nickel.

3. Composition catalytique selon l'une des revendications 1 ou 2 dans laquelle l'halogénure de lithium est le chlorure de lithium.

4. Composition catalytique selon l'une des revendications 1 ou 2 dans laquelle l'halogénure de lithium est le bromure de lithium.

5. Composition catalytique selon l'une des revendications précédentes dans laquelle le composé de l'aluminium est choisi dans le groupe constitué par les chlorures d'alkylaluminium.

6. Composition catalytique selon l'une des revendications précédentes dans laquelle le composé de l'aluminium est le dichloroéthylaluminium.

7. Composition catalytique selon l'une des revendications précédentes dans laquelle le rapport molaire entre l'halogénure d'hydrocarbylaluminium et l'halogénure de lithium est supérieure à 1.

8. Composition catalytique selon l'une des revendications 2 à 7, dans laquelle le composé du nickel est un sel de nickel.

9. Composition catalytique selon l'une des revendications 2 à 8, dans laquelle le composé du nickel est un complexe zérovalent, monovalent ou divalent.

10. Composition catalytique selon l'une des revendications 2 à 9, dans laquelle les composés de nickel sont choisis dans le groupe formé par les halogénures, le sulfate, le phosphate, les carboxylates, les acétylacétonates, les tétrahalogénoborates et les complexes que font ces sels avec les amines, les phosphines, les ethers, les nitriles, les esters, seuls ou en mélange.

11. Composition catalytique selon l'une des revendications 2 à 10, dans laquelle le composé de nickel est NiCl₂, NiCl₂.2Pyridine, NiCl₂.2P(i-Pr)₃, NiCl_{2.}2P(Bu)₃, Ni(acétonitrile)₆(BF₄)₂, le 2-éthylhexanoate de nickel, l'acétylacétonate de nickel.

12. Composition catalytique selon l'une des revendications précédentes résultant du mélange d'au moins un composé d'un élément catalytique avec le mélange liquide formé par au moins un halogénure de lithium et au moins un halogénure d'hydrocarbylaluminium.

13. Composition catalytique selon l'une des revendications précédentes dans laquelle la concentration du (des) composé(s) de l'élément catalytique dans le mélange liquide formé par au moins un halogénure de lithium et au moins un halogénure d'hydrocarbylauminium est compris entre 2 et 200 mmoles par litre.

14. Composition selon l'une des revendications 12 ou 13 dans laquelle l'élément est le nickel.

15. Composition catalytique solide obtenue à partir du mélange liquide d'au moins un halogénure de lithium avec au moins un halogénure d'hydrocarbylaluminium, puis introduction d'au moins une phase hydrocarbonée et d'au moins un élément catalytique, ledit catalyseur solide se séparant de la phase organique liquide.

16. Composition catalytique solide selon la revendication 15 dans laquelle la phase hydrocarbonée contient au moins un réactif et/ou au moins un produit de la réaction.

17. Composition catalytique solide selon l'une quelconque des revendications 15 et 16 dans laquelle l'élément catalytique est le nickel.

18. Composition catalytique solide selon l'une quelconque des revendications 16 à 17, obtenue à partir du mélange liquide d'au moins un halogénure de lithium avec au moins un halogénure d'hydrocarbylaluminium, puis addition d'au moins un composé de l'élément catalytique et introduction d'une phase hydrocarbonée avant, pendant ou après l'addition dudit composé du nickel, ledit catalyseur solide se séparant de la phase liquide hydrocarbonée.

19. Procédé pour la dimérisation, la codimérisation, l'oligomérisation, la cooligomérisation des oléfines à l'aide d'une composition catalytique selon l'une quelconque des revendications 1 à 18.

20. Procédé selon la revendication 19 dans lequel l'oléfine est le propylène

21. Procédé selon la revendication 19 dans lequel l'oléfine est le butène.

22. Procédé selon l'une des revendications 19 à 21 dans lequel la réaction est conduite de façon continue ou semi continue et dans lequel la phase hydrocarbonée contenant les dimères, les codimères, les oligomères ou les cooligomères, séparée de la phase solide contenant le catalyseur est extraite.

23. Procédé selon l'une des revendications 19 à 22, opérant entre -30 et 100°C, sous une pression maintenant au moins en partie la et/ou les oléfines en phase liquide.

## Claims

1. A catalytic composition comprising at least one lithium halide, at least one hydrocarbylaluminium halide and at least one compound of a catalytic element.

2. A composition according to claim 1, in which the compound is a nickel compound.

3. A catalytic composition according to claim 1 or claim 2, in which the lithium halide is lithium chloride.

4. A catalytic composition according to claim 1 or claim 2, in which the lithium halide is lithium bromide.

5. A catalytic composition according to any one of the preceding claims, in which the hydrocarbonaluminium halide is selected from the group constituted by alkylaluminium chlorides.

6. A catalytic composition according to any one of the preceding claims, in which the aluminium compound is dichloroethylaluminium.

7. A catalytic composition according to any one of the preceeding claims, in which the molar ratio between the hydrocarbylaluminium halide and the lithium halide is greater than 1.

8. A catalytic composition according to any one of the claims 2 to 7, in which the nickel compound is a nickel salt.

9. A catalytic composition according to any one of the claims 2 to 8, in which the nickel compound is a zerovalent, monovalent or divalent complex.

10. A catalytic composition according to any one of the claims 2 to 9, in which the nickel compound is selected from the group formed by the halides, the sulphate, the phosphate, the carboxylates, the acetylacetonates, the tetrahalogenoborates and complexes which form salts with amines, phosphines, ethers, nitriles and esters, either alone or as a mixture.

11. A catalytic composition according to any one of the claims 2 to 10, in which the nickel compound is NiCl₂, NiCl₂.2Pyridine, NiCl₂.2P(i-Pr)₃, NiCl₂.2P(Bu)₃, Ni(acetonitrile)₆(BF₄)₂, nickel 2-ethylhexanoate or nickel acetylacetonate.

12. A catalytic composition according to any one of the preceding claims, resulting from mixing at least one compound of a catalytic element with the liquid mixture formed by at least one lithium halide and at least one hydrocarbylaluminium halide.

13. A catalytic composition according to any one of the preceding claims, in which the concentration of the compound(s) of the catalytic element in the liquid mixture formed by at least one lithium halide and at least one hydrocarbylaluminium halide is in the range 2 mmoles per litre to 200 mmoles per litre.

14. A composition according to claim 12 or claim 13, in which the element is nickel.

15. A solid catalytic composition obtained from a liquid mixture of at least one lithium halide with at least one hydrocarbylaluminium halide, then the introduction of at least one hydrocarbon phase and at least one catalytic element, said solid catalyst separating from the liquid organic phase.

16. A solid catalytic composition according to claim 15, in which the hydrocarbon phase contains at least one reactant and/or at least one reaction product.

17. A catalytic composition according to claim 15 or claim 16, in which the catalytic element is nickel.

18. A solid catalytic composition according to claim 16 or claim 17, obtained from a liquid mixture of at least one lithium halide with at least one hydrocarbylaluminium halide, plus the addition of at least one compound of a catalytic element and introduction of a hydrocarbon phase before, during or after addition of the nickel, said solid catalyst separating from the liquid hydrocarbon phase.

19. A process for the dimerisation, codimerisation, oligomerisation or co-oligomerisation of olefins using a catalytic composition according to any one of claims 1 to 18.

20. A process according to claim 19, in which the olefin is propylene.

21. A process according to claim 19, in which the olefin is butene.

22. A process according to any one of claims 19 to 21, in which the reaction is carried out continuously or semi-continously and in which the hydrocarbonated phase containing dimers, codimers, oligomers or co-oligomers, is separate from the solid phase containing the catalyst, is extracted.

23. A process according to any one of claims 19 to 22, working between -30°C and 100°C, under a pressure such as at least a part of the olefin(s) is maintained in the liquid phase.

## Patentansprüche

1. Katalytische Zusammensetzung, wenigstens ein Lithiumhalogenid, wenigstens ein Hydrocarbylamluminiumhalogenid und wenigstens eine Verbindung eines katalytischen Elements umfassend.

2. Zusammensetzung nach Anspruch 1, bei der die Verbindung eine Nickelverbindung ist.

3. Katalytische Zusammensetzung nach einem der Ansprüche 1 oder 2, bei der das Lithiumhalogenid Lithiumchlorid ist.

4. Katalytische Zusammensetzung nach einem der Ansprüche 1 oder 2, bei dem das Lithiumhalogenid Lithiumbromid ist.

5. Katalytische Zusammensetzung nach einem der vorhergenden Ansprüche, bei der die Aluminiumverbindung gewählt ist aus der Gruppe, die durch die Alkylaluminiumchloride gebildet ist.

6. Katalytische Zusammensetzung nach einem der vorhergehenden Ansprüche, bei der die Aluminiumverbindung Dichlorethylaluminium ist.

7. Katalytische Zusammensetzung nach einem der vorhergehenden Ansprüche, bei der das Molverhältnis zwischen dem Hydrocarbylaluminiumhalogenid und dem Lithiumhalogenid größer als 1 ist.

8. Katalytische Zusammensetzung nach einem der Ansprüche 2 bis 7, bei der die Nickelverbindung ein Nickelsalz ist.

9. Katalytische Zusammensetzung nach einem der Ansprüche 2 bis 8, bei der die Nickelverbindung ein nullwertiger, einwertiger oder zweiwertiger Komplex ist.

10. Katalytische Zusammensetzung nach einem der Ansprüche 2 bis 9, bei der die Nickelverbindungen gewählt sind aus der durch die Halogenide, das Sulfat, das Phosphat, die Karboxilate, die Acylacetonate, die Tetrahalogenborate und die Komplexe, welche diese Salze mit den Aminen, den Phosphinen, den Äthern, den Nitrilen, den Estern, allein oder im Gemisch formen, gebildeten Gruppe.

11. Katalytische Zusammensetzung nach einem der Ansprüche 2 bis 10, bei der die Nickelverbindung NiCL₂, NiCl₂.2pyridin, NiCl₂.2P(i-Pr)₃, NiCl₂.2P(Bu)₃, Ni(Acetonnitril)₆(BF₄)₂, Nickel-2-Ethylhexanoat oder Nickelacetylacetonat ist.

12. Katalytische Zusammensetzung nach einem der vorhergenden Ansprüche resultierend aus dem Gemisch wenigstens einer Verbindung eines katalytischen Elements mit dem flüssigen Gemisch, das gebildet wird durch wenigstens ein Lithiumhalogenid und wenigstens ein Hydrocarbylaluminiumhalogenid.

13. Katalytische Zusammensetzung nach einem der vorhergehenden Ansprüche, bei der die Konzentration des (der) Verbindung(en) des katalytischen Elements im flüssigen durch wenigstens ein Lithiumhalogenid und wenigstens ein Hydrocarbylaluminiumhalogenid geformten Gemisch zwischen 2 und 200 mMol/l beträgt.

14. Zusammensetzung nach einem der Ansprüche 12 oder 13, bei der das Element Nickel ist.

15. Katalytische Feststoffzusammensetzung, erhalten aus dem flüssigen Gemisch wenigstens eines Lithiumhalogenids mit wenigstens einem Hydrocarbylaluminiumhalogenid, dann Einführen wenigstens einer kohlenstoffhaltigen Phase und wenigstens eines katalytischen Elements, wobei dieser feste Katalysator sich von der organischen flüssigen Phase trennt.

16. Katalytische Zusammensetzung nach Anspruch 15, bei der die kohlenwasserstoffhaltige Phase wenigstens ein Reaktionsmittel und/oder wenigstens ein Reaktionsprodukt enthält.

17. Katalytische Feststoffzusammensetzung nach Ansprüchen 15 und 16, bei der das katalytische Element Nickel ist.

18. Katalytische Feststoffzusammensetzung nach einem der Ansprüche 16 bis 17, erhalten aus dem flüssigen Gemisch wenigstens eines Lithiumhalogenids mit wenigstens einem Hydrocarbylaluminiumhalogenid, dann Zugabe wenigstens einer Verbindung des katalytischen Elements und Einführen einer kohlenwasserstoffhaltigen Phase vor, während oder nach Zugabe dieser Nickelverbindung, wobei der feste Katalysator sich von der kohlenwasserstoffhaltigen flüssigen Phase trennt.

19. Verfahren zur Dimerisierung, Codimerisierung, Oligomerisierung, Cooligomerisierung der Olefine mit Hilfe einer katalytischen Verbindung nach einem der Ansprüche 1 bis 18.

20. Verfahren nach Anspruch 19, bei dem das Olefin Propylen ist.

21. Verfahren nach Anspruch 19, bei dem das Olefin Buten ist.

22. Verfahren nach einem der Ansprüche 19 bis 21, bei dem die Reaktion kontinuierlich oder halbkontinuierlich durchgeführt wird und bei dem die kohlenwasserstoffhaltige, die Dimere, die Kodimere und die Oligomere oder die Cooligomere enthaltende Phase getrennt von der festen, den Katalysator enthaltenden Phase, extrahiert wird.

23. Verfahren nach einem der Ansprüche 19 bis 22, durchgeführt zwischen -30 und 100°C, unter einem Druck, der wenigstens zum Teil das oder die Olefine in flüssiger Phase hält.
